# EUROPEAN PATENT APPLICATION

(11) **EP 4 659 745 A1**
(43) Date of publication of application: **10.12.2025**
(21) Application number: 24749699.5
(22) Date of filing: 31.01.2024
(51) Int. Cl.: A61K 9/22, A61K 31/4015, A61K 47/32, A61K 47/38, A61K 47/26, A61K 47/02, A61K 47/04, A61K 47/12, A61K 47/44, A61K 47/36, A61P 25/08

(54) **BRIVARACETAM DOUBLE-RELEASE THREE-LAYER TABLET AND PREPARATION METHOD THEREFOR**

(30) Priority: 02.02.2023 CN 202310094041
(71) Applicant: Taizhou Overseas Pharmaceuticals, Ltd., Taizhou, Jiangsu 225316 (CN)
(72) Inventor: YE, Xiaomei, Taizhou, Jiangsu 225316 (CN); PAN, Yongjian, Taizhou, Jiangsu 225316 (CN)
(74) Representative: Murgitroyd & Company
(86) International application number: PCT/CN2024/074852
(87) International publication number: WO 2024/160220

(57) **Abstract**

The present disclosure relates to a double-release three-layered brivaracetam tablet and a preparation method thereof, belonging to pharmaceutical technology, which is characterized in that the tablet is made of an immediate-release layer, a retarding layer and a sustained-release layer that are superposed; where the retarding layer contains no drug and is located in the middle; at least 75% of an surface area of each of the immediate-release layer and the sustained-release layer which is in contact with the retarding layer is covered by the retarding layer, and a sustained-release material in the retarding layer contains a waxy sustained-release material; and the tablet has the following characteristics: when tested using the paddle method at 50 rpm in media of pH 1.2, pH 4.5, pH 6.8, or water, not less than 25% of the API is released within 30 minutes, 30-65% within 2 hours, not less than 65% within 6 hours, and not less than 80% but less than 100% within 14 hours. After 30 minutes, the release follows a zero-order kinetic profile. This formulation significantly improves the release characteristics of brivaracetam, enhances medication compliance and safety, and optimizes its therapeutic effect.

## Description

### TECHNICAL FIELD

The present disclosure relates to pharmaceutical technology, and in particular to a double-release three-layered brivaracetam tablet and a preparation method thereof.

### BACKGROUND

Brivaracetam, with the chemical name (2S)-2-[(4R)-2-oxo-4-propyltetrahydro-1H-pyrrol-1-yl]butanamide and the molecular formula C₁₁H₂₀N₂O₂, is an effective antiepileptic drug. Clinically, it is used as monotherapy for the treatment of partial-onset seizures in patients aged 4 years and older. Brivaracetam is a weakly acidic liquid with good water solubility, but it has a relatively short duration of effective plasma concentration in vivo. Currently marketed brivaracetam tablets and oral solutions require twice-daily administration.

Among existing reports on the release behavior of brivaracetam, publications such as CN106692095A, CN201510202995, and CN114028343A disclose immediate-release formulations of brivaracetam. These formulations share the same disadvantages as currently marketed products, including excessively rapid drug release, large fluctuations in plasma drug concentration, poor controllability, pronounced adverse reactions, significant toxicity, frequent dosing, and poor patient compliance. On the other hand, CN113908153A and CN102046153B disclose sustained-release formulations of brivaracetam. Although these formulations provide prolonged release, they suffer from a slow onset of action and limited therapeutic efficacy.

### SUMMARY

In order to improve the release characteristics of the drug brivaracetam, improve medication compliance and safety, and optimize its therapeutic efficacy, the present disclosure provides a brivaracetam tablet and method for its preparation.

In a first aspect of the present disclosure, a double-release three-layered brivaracetam tablet is provided, which is made of an immediate-release layer, a retarding layer and a sustained-release layer that are superposed; wherein
the retarding layer contains no drug and is located in the middle;
at least 60% of the surface area of each of the immediate-release layer and the sustained-release layer that is in contact with the retarding layer is covered by the retarding layer; and
a sustained-release material in the retarding layer contains a waxy sustained-release material;
drug substance is selected from one or more of brivaracetam, a pharmaceutically acceptable complex thereof, a pharmaceutically acceptable salt thereof, a pharmaceutically acceptable solvate thereof, a pharmaceutically acceptable hydrate thereof, and other related structural derivatives of levetiracetam;
the tablet has the following characteristics: when tested using the paddle method at 50 rpm, in medium of pH 1.2, pH 4.5, pH 6.8, or water in an aqueous medium, a dissolution rate of the drug substance is not less than 25% at 0.5 hour, from 30% to 65% at 2 hours, not less than 65% at 6 hours, and not less than 80% but less than 100% at 14 hours; and after 0.5 hour, the tablet is released in vitro in a zero-order release manner

Preferably, the double-release three-layered brivaracetam tablet is characterized in that the content of the drug substance in the immediate-release layer accounts for 20 to35%, or 25 to30% of the total amount of the drug substance; and the content of the drug substance in the sustained-release layer account for 65-80% or 70-75% of the total amount of the drug substance

Preferably, the double-release three-layered brivaracetam tablet is characterized in that, the formulation of the immediate-release layer comprises:
a disintegrant in an amount of 2% to 7.5%, or preferably 5%, by weight of the immediate-release layer, wherein the disintegrant is selected from one or more of crosslinked sodium carboxymethyl starch, crosslinked sodium carboxymethyl cellulose, cross-linked povidone, and low-substituted hydroxypropyl cellulose;
this selection ensures that an immediate-release portion is released quickly and takes effect in the gastrointestinal environment;
a filler in an amount of 40% to 85% by weight of the immediate-release layer, wherein the filler is selected from one or more of lactose, microcrystalline cellulose, anhydrous dibasic calcium phosphate, and corn starch; and preferably, the filler is a combination of anhydrous calcium hydrogen phosphate and corn starch, or a combination of microcrystalline cellulose and lactose.

Preferably, wherein in the combination of microcrystalline cellulose and lactose, the weight ratio of the two components is from 1:1 to 1:3. This selection ensures that the granules of the immediate-release layer have good compressibility and fluidity, and tablets with good consistency are obtained; an adhesive in an amount of 3% to 8% by weight of the immediate-release layer, wherein the adhesive is selected from hydroxypropyl cellulose and an aqueous solution of povidone K30.

Preferably, the waxy sustained-release material is selected from one or more of glyceryl behenate, carnauba wax and cetyl alcohol.

Preferably, the sustained-release material in the retarding layer consists of a waxy sustained-release material and a swelling-type sustained-release material, wherein the weight ratio of the waxy sustained-release material to the swelling-type sustained-release material is 0.4 to 0.8 or 0.5 to 0.7; and the combined content of the two materials accounts for not less than 60% by weight of the formulation of the retarding layer;

The swelling-type sustained-release material is one or more selected from guar gum, vegetable gum, seaweed glue, hydroxypropyl methylcellulose, hydroxymethyl cellulose and hydroxyethyl cellulose.

Preferably, the retarding layer has a thickness greater than or equal to 1 mm; and preferentially the thickness is 1-3 mm.

Preferably, the filler is present in an amount of 15% to 30%, preferably 20% to 27%, by weight of the retarding layer; and lactose, microcrystalline cellulose, anhydrous calcium hydrogen phosphate or mannitol is selected as the filler; and microcrystalline cellulose and lactose are preferred. Hydroxypropyl cellulose or a povidone K30 aqueous solution is selected as the adhesive, and the adhesive accounts for 6%-15% of the formulation amount of the retarding layer, so as to ensure proper flowability in the preparation process.

Preferably, a formulation of the sustained-release layer includes an active ingredient, a sustained-release material, a filler, a flow aid and a lubricant.

The sustained-release material is present in an amount of20-60%, and preferably 25-40% by weight of the formulation. Any one or two of HPMC K15M, HPMC K4M, HPMC K750, HPMC K100M, HPMC K100LV, EUDRAGIT^{®} L100-55, glyceryl behenate and xanthan gum is adopted as the sustained-release material, and a combination of HPMC K100M and EUDRAGIT^{®} L100-55, or a combination of HPMC K4M and EUDRAGIT^{®} L100-55, or HPMC K100M is further preferred.

The filler is present in an amount of 35% -65% by weight of the sustained-release layer, and one or more of microcrystalline cellulose, lactose, anhydrous calcium hydrogen phosphate, corn starch and the like is adopted as the filler. Through research, the present invention has been found that the parameter selection ensures compressibility and fluidity as well as sufficient porosity after tableting.

Any of the aforementioned double-release three-layered brivaracetam tablets also has a film coating, the coating material being a gastric-soluble coating powder, and the weight gain from the film coating is 2% to 5%, preferably 3%.

In any of the aforementioned double-release three-layered brivaracetam tablets, in the formulation of the immediate-release layer or the formulation of the sustained-release layer, the content of the drug substance is no higher than 20%. Through research, in the present disclosure it is found that the adoption of this parameter can ensure that the drug substance is fully dispersed by an accessory material, and can effectively avoid pressure dissolution and sticking in the tableting process due to a low melting point of API.

On the other hand, the present disclosure provides a method for preparing any one of the aforementioned double-release three-layered brivaracetam tablets, comprising the following steps:
step a. weigh the brivaracetam, disintegrant, and filler in amounts required for the formulation of the immediate-release layer, and mix them thoroughly; add an adhesive to the mixture and perform wet granulation, after wet sizing, dry the granules until the moisture content is less than 4.0%, then carry out dry sizing and final blending to obtain the final granules of the immediate-release layer;
step b. weigh a sustained-release material and a filler in amounts required for the formulation of a sustained-release layer, and mix them uniformly; add a proper amount of purified water by spray addition for wet granulation, after wet sizing, dry the granules until the moisture content is less than 4.0%, then perform dry sizing and final blending to obtain the final granules of the sustained-release layer; and
step c. weigh a sustained-release material and a filler in amounts required for the formulation of the retarding layer, and mix them uniformly; add an adhesive, and perform wet granulation. After wet sizing, dry the granules until the moisture content is less than 4.0%, then perform dry sizing and final blending to obtain the final granules of the retarding layer.

Further, the final blending in the Step a comprises: mixing a portion of the dried granules with a glidant in amounts required for the formulation, followed by sieving; pre-mixing the resulting blend with the remaining dried granules of the immediate-release layer; then mixing a portion of the pre-mixture with a lubricant in amounts required for the formulation, followed by sieving; and then blending with the remaining pre-mixture to obtain the final granules of the immediate-release;

Further, the final blending in the Step b comprises: mixing a portion of the dried granules with a glidant in amounts required for the formulation, followed by sieving; pre-mixing the resulting blend with the remaining dried granules of the sustained-release layer; mixing a portion of the pre-mixture a lubricant in amounts required for the formulation, followed by sieving; and then blending with the remaining pre-mixture to obtain the final granules of the sustained-release layer.Further, the final blending in the step c comprises: mixing a portion of the dried granules with glidant in amounts required for the formulation, followed by sieving; pre-mixing the resulting blend with the remaining dried granules of the retarding layer; mixing a portion of the pre-mixture with a lubricant in amounts required for the formulation, followed by sieving; and then blending with the remaining pre-mixture to obtain the final granules of the retarding layer. Preferably, it further includes a tableting step, and a tableting sequence is the sustained-release layer, the retarding layer and the immediate-release layer.

Compared with the prior art, the technical solution of the present disclosure has the following advantages.
1. Structurally, the tablet comprises both an immediate-release portion and a sustained-release portion, enabling it to achieve an effect of rapid onset of action of the existing immediate-release preparation of brivaracetam and while also providing the prolonged, steady release typical of conventional sustained-release formulations. This dual-release profile reduces the frequency of administration, improves patient compliance, and lowers the incidence of adverse effects caused by excessive fluctuations in plasma drug concentration.
   Compared with the existing technology of the sustained-release preparation of brivaracetam, the present invention develops a dedicated retarding layer formulation, positioned between the immediate-release layer and the sustained-release layer. This configuration reduces one release surface of the sustained-release layer compared to conventional sustained-release formulations. The retarding layer ensures a more stable release from the sustained-release layer, enabling it to achieve a zero-order release profile..
2. In terms of API distribution, the present invention adopts an immediate-release to sustained-release API ratio of 20%-35% to 65%-80%, which helps avoid the adverse effects caused by excessively high peak plasma concentrations associated with existing brivaracetam immediate-release formulations, while also overcoming the drawback of delayed onset of action seen in current brivaracetam sustained-release tablet technologies.
3. The API content in both the immediate-release layer and the sustained-release layer does not exceed 20%, ensuring that the API is adequately dispersed within the excipients. This effectively prevents issues such as sticking and picking during tableting caused by the low melting point of the API and pressure-induced dissolution, thereby maintaining tablet weight uniformity and consistent dissolution profiles..

In summary, the test results demonstrate that the tablets provided by the present invention achieve not less than 25% API dissolution within 30 minutes, 30-60% within 2 hours, not less than 60% within 6 hours, and not less than 80% but less than 100% within 14 hours. After 30 minutes of administration, the dissolution follows a zero-order kinetic profile, showing excellent consistency in dissolution and tablet weight. The formulation effectively combines rapid onset of action with prolonged and steady drug release, enabling once-daily dosing to achieve therapeutic efficacy.

### BRIEF DESCRIPTION OF DRAWINGS

FIG. 1 shows tablet structures of Examples 1-7 and Comparative Examples 1-8;
FIG. 2 is a dissolution chart of brivaracetam in Example 1 and Comparative Example 1;
FIG. 3 is a dissolution chart of brivaracetam in Example 2 and Comparative Example 2;
FIG. 4 is a diagram showing dissolution deviations of brivaracetam in Example 3 and Comparative Example 3;
FIG. 5 is a dissolution chart of brivaracetam in Example 4 and Comparative Example 5;
FIG. 6 is a dissolution chart of brivaracetam in Example 4 and Comparative Example 6;
FIG. 7 is a dissolution chart of brivaracetam in Example 5 and Comparative Example 7.

### DETAILED DESCRIPTION

The technical solution of the present disclosure will be described in detail hereafter with reference to exemplary embodiments, but it should not be understood as limiting the claimed scope of the present disclosure.

### Examples 1-7. Exemplary formulations of the present disclosure

The formulations corresponding to Examples 1-7 were shown in Tables 1-7, and a preparation method included the following steps:
**1. Preparation** of total blended granules of immediate-release layer, total blended granules of sustained-release layer, and total blended granules of retarding layer:
   Step a. weigh the brivaracetam, disintegrant, and filler in amounts required for the formulation of the immediate-release layer, and mix them thoroughly; add an adhesive to the mixture and perform wet granulation, after wet sizing, dry the granules until the moisture content is less than 4.0%, then carry out dry sizing and final blending to obtain the final granules of the immediate-release layer.
      Wherein the final blending comprises mixing a portion of the dried granules with a glidant in amounts required for the formulation, followed by sieving; pre-mixing the resulting blend with the remaining dried granules of the immediate-release layer; then mixing a portion of the pre-mixture with a lubricant in amounts required for the formulation, followed by sieving; and then blending with the remaining pre-mixture to obtain the final granules of the immediate-release.
   Step b. weigh a sustained-release material and a filler in amounts required for the formulation of a sustained-release layer, and mix them uniformly; add a proper amount of purified water by spray addition for wet granulation, after wet sizing, dry the granules until the moisture content is less than 4.0%, then perform dry sizing and final blending to obtain the final granules of the sustained-release layer.
      Wherein the final blending in the Step b comprises: mixing a portion of the dried granules with a glidant in amounts required for the formulation, followed by sieving; pre-mixing the resulting blend with the remaining dried granules of the sustained-release layer; mixing a portion of the pre-mixture a lubricant in amounts required for the formulation, followed by sieving; and then blending with the remaining pre-mixture to obtain the final granules of the sustained-release layer;
   Step c. weigh a sustained-release material and a filler in amounts required for the formulation of the retarding layer, and mix them uniformly; add an adhesive, and perform wet granulation. After wet sizing, dry the granules until the moisture content is less than 4.0%, then perform dry sizing and final blending to obtain the final granules of the retarding layer;
      Wherein the final blending in the step c comprises: mixing a portion of the dried granules with glidant in amounts required for the formulation, followed by sieving; pre-mixing the resulting blend with the remaining dried granules of the retarding layer; mixing a portion of the pre-mixture with a lubricant in amounts required for the formulation, followed by sieving; and then blending with the remaining pre-mixture to obtain the final granules of the retarding layer.

### 2. Tableting

The tableting sequence of the three-layered tablet might be: the sustained-release layer > the retarding layer > the immediate-release layer, the immediate-release layer > the retarding layer > the sustained-release layer, the retarding layer > the sustained-release layer > the immediate-release layer.

It was found through test that the hardness consistency and dissolution consistency of the in vitro dissolution curve of the three-layered tablet obtained by tableting in the order of the sustained-release layer > the retarding layer > the immediate-release layer were good.

**Table 1. Formulation of Example 1**

| Layes of tablet | Material | Example 1 | |
|---|---|---|---|
| | | % ratio (w/w) in formulation of layer | Unit formulation amount (mg/tablet) |
| Immediate-release layer | Brivaracetam | 8.18 | 18 |
| | Cross-linked sodium carboxylmethyl cellulose | 4.55 | 10 |
| | Lactose | 54.55 | 120 |
| | Anhydrous calcium hydrogen phosphate | 25.00 | 55 |
| | Povidone K30 | 6.00 | 13.2 |
| | Magnesium Stearate | 1.14 | 2.5 |
| | Colloidal Silica | 0.59 | 1.3 |
| Sustained-release layer | Brivaracetam | 18.67 | 40 |
| | HPMC K100M | 21.00 | 45 |
| | EUDRAGIT^{®} L100-55 | 6.07 | 13 |
| | Anhydrous calcium hydrogen phosphate | 26.13 | 56 |
| | Microcrystalline cellulose PH101 | 26.60 | 57 |
| | Colloidal Silica | 0.47 | 1 |
| | Magnesium Stearate | 1.07 | 2.3 |
| Retarding layer | HPMC K4M | 44.12 | 75 |
| | Lactose | 22.35 | 38 |
| | Carnauba wax | 24.12 | 41 |
| | Povidone K30 | 7.35 | 12.5 |
| | Colloidal Silica | 1.06 | 1.8 |
| | Magnesium Stearate | 1.00 | 1.7 |
| Film coating | Opadry^{®} II 85F38197-CN Yellow | 100.00 | 18.6 |

**Table 2. Formulation of Example 2**

| Layes of tablet | Raw accessory material | Example **2** | |
|---|---|---|---|
| | | % ratio (w/w) in the formulation of layer | Unit formulation amount (mg/tablet) |
| Immediate-release layer | Brivaracetam | 5.45 | 12 |
| | Cross-linked carboxymethyl starch sodium | 2.00 | 4.4 |
| | Corn starch | 52.27 | 115 |
| | Anhydrous calcium hydrogen phosphate | 30.91 | 68 |
| | Hydroxy propyl cellulose | 7.32 | 16.1 |
| | Sodium stearyl fumarate | 1.36 | 3 |
| | Colloidal Silica | 0.68 | 1.5 |
| Sustained-release layer | Brivaracetam | 17.04 | 38 |
| | HPMC K100M | 32.29 | 72 |
| | Anhydrous calcium | 26.77 | 59.7 |
| | hydrogen phosphate | | |
| | Corn starch | 22.33 | 49.8 |
| | Colloidal Silica | 0.54 | 1.2 |
| | Sodium stearyl fumarate | 1.03 | 2.3 |
| Retarding layer | Guar gum | 47.06 | 80 |
| | Anhydrous calcium hydrogen phosphate | 20.59 | 35 |
| | Carnauba wax | 23.53 | 40 |
| | Hydroxy propyl cellulose | 6.71 | 11.4 |
| | Colloidal Silica | 1.12 | 1.9 |
| | Sodium stearyl fumarate | 1.00 | 1.7 |
| Film coating | Opadry^{®} II 85F38197-CN Yellow | 100.00 | 18.6 |

**Table 3. Formulation of Example 3**

| Layes of tablet | Raw accessory material | Example **3** | |
|---|---|---|---|
| | | % ratio (w/w) in the formulation of layer | Unit formulation amount (mg/tablet) |
| Immediate-release layer | Brivaracetam | 8.18 | 18 |
| | Cross-linked carboxymethyl starch sodium | 5.00 | 11 |
| | Corn starch | 54.41 | 119.7 |
| | Anhydrous calcium hydrogen phosphate | 25.91 | 57 |
| | Hydroxy propyl cellulose | 5.00 | 11 |
| | Sodium stearyl fumarate | 1.00 | 2.2 |
| | Colloidal Silica | 0.50 | 1.1 |
| Sustained-release layer | Brivaracetam | 19.70 | 52 |
| | HPMC K4M | 22.10 | 58.35 |
| | EUDRAGIT^{®} L100-55 | 14.94 | 39.45 |
| | Anhydrous calcium hydrogen phosphate | 19.09 | 50.4 |
| | Corn starch | 22.86 | 60.35 |
| | Colloidal Silica | 0.44 | 1.15 |
| | Sodium stearyl fumarate | 0.87 | 2.3 |
| Retarding layer | Guar gum | 42.41 | 72.1 |
| | Anhydrous calcium hydrogen phosphate | 21.18 | 36 |
| | Carnauba wax | 24.41 | 41.5 |
| | Hydroxy propyl cellulose | 10.00 | 17 |
| | Colloidal Silica | 1.00 | 1.7 |
| | Sodium stearyl fumarate | 1.00 | 1.7 |
| Film coating | Opadry^{®} II 85F38197-CN Yellow | 100.00 | 18.6 |

**Table 4. Formulation of Example 4**

| Layes of tablet | Raw accessory material | Example **4** | |
|---|---|---|---|
| | | % ratio (w/w) in the formulation of layer | Unit formulation amount (mg/tablet) |
| Immediate-release layer | Brivaracetam | 12.84 | 28 |
| | Cross-linked carboxymethyl starch sodium | 6.88 | 15 |
| | Corn starch | 46.24 | 100.8 |
| | Anhydrous calcium hydrogen phosphate | 28.58 | 62.3 |
| | Hydroxy propyl cellulose | 3.94 | 8.6 |
| | Sodium stearyl fumarate | 1.01 | 2.2 |
| | Colloidal Silica | 0.50 | 1.1 |
| Sustained-release layer | Brivaracetam | 16.11 | 72 |
| | HPMC K100M | 20.13 | 90 |
| | EUDRAGIT^{®} L100-55 | 6.71 | 30 |
| | Anhydrous calcium hydrogen phosphate | 25.77 | 115.2 |
| | Corn starch | 29.17 | 130.4 |
| | Colloidal Silica | 1.07 | 4.8 |
| | Sodium stearyl fumarate | 1.03 | 4.6 |
| Retarding layer | HPMC K4M | 35.88 | 61 |
| | Anhydrous calcium hydrogen phosphate | 26.06 | 44.3 |
| | Carnauba wax | 28.24 | 48 |
| | Hydroxy propyl cellulose | 8.41 | 14.3 |
| | Colloidal Silica | 0.71 | 1.2 |
| | Sodium stearyl fumarate | 0.71 | 1.2 |
| Film coating | Opadry^{®} II 85F38197-CN Yellow | 100.00 | 25.5 |

**Table 5. Formulation of Example 5**

| Layes of tablet | Raw accessory material | Example 5 | |
|---|---|---|---|
| | | % ratio (w/w) in the formulation of layer | Unit formulation amount (mg/tablet) |
| Immediate-release layer | Brivaracetam | 11.31 | 25 |
| | Cross-linked carboxymethyl starch sodium | 4.52 | 10 |
| | Corn starch | 52.49 | 116 |
| | Anhydrous calcium hydrogen phosphate | 26.24 | 58 |
| | Hydroxy propyl cellulose | 3.44 | 7.6 |
| | Sodium stearyl fumarate | 1.13 | 2.5 |
| | Colloidal Silica | 0.86 | 1.9 |
| Sustained-release layer | Brivaracetam | 15.81 | 68 |
| | HPMC K4M | 20.70 | 89.025 |
| | EUDRAGIT^{®} L100-55 | 16.20 | 69.675 |
| | Anhydrous calcium hydrogen phosphate | 20.53 | 88.3 |
| | Corn starch | 24.74 | 106.4 |
| | Colloidal Silica | 0.74 | 3.2 |
| | Sodium stearyl fumarate | 1.26 | 5.4 |
| Retarding layer | HPMC K4M | 41.18 | 70 |
| | Anhydrous calcium hydrogen phosphate | 23.53 | 40 |
| | Glyceryl behenate | 26.47 | 45 |
| | Hydroxy propyl cellulose | 7.71 | 13.1 |
| | Colloidal Silica | 0.53 | 0.9 |
| | Sodium stearyl fumarate | 0.59 | 1 |
| Film coating | Opadry^{®} II 85F38197-CN Yellow | 100.00 | 25.5 |

**Table 6. Formulation of Example 6**

| Layes of tablet | Raw accessory material | Example **6** | |
|---|---|---|---|
| | | % ratio (w/w) in the formulation of layer | Unit formulation amount (mg/tablet) |
| Immediate-release layer | Brivaracetam | 11.31 | 25 |
| | Cross-linked carboxymethyl starch sodium | 4.52 | 10 |
| | Corn starch | 39.37 | 87 |
| | Anhydrous calcium hydrogen phosphate | 39.37 | 87 |
| | Hydroxy propyl cellulose | 3.44 | 7.6 |
| | Sodium stearyl fumarate | 1.13 | 2.5 |
| | Colloidal Silica | 0.86 | 1.9 |
| Sustained-release layer | Brivaracetam | 15.81 | 68 |
| | HPMC K4M | 20.70 | 89.025 |
| | EUDRAGIT^{®} L100-55 | 16.20 | 69.675 |
| | Anhydrous calcium hydrogen phosphate | 20.53 | 88.3 |
| | Corn starch | 24.74 | 106.4 |
| | Colloidal Silica | 0.74 | 3.2 |
| | Sodium stearyl fumarate | 1.26 | 5.4 |
| Retarding layer | HPMC K4M | 41.18 | 70 |
| | Anhydrous calcium hydrogen phosphate | 23.53 | 40 |
| | Glyceryl behenate | 26.47 | 45 |
| | Hydroxy propyl cellulose | 7.71 | 13.1 |
| | Colloidal Silica | 0.53 | 0.9 |
| | Sodium stearyl fumarate | 0.59 | 1 |
| Film coating | Opadry^{®} II 85F38197-CN Yellow | 100.00 | 25.5 |

**Table 7. Formulation of Example 7**

| Layes of tablet | Raw accessory material | Example 5 | |
|---|---|---|---|
| | | % ratio (w/w) in the formulation of layer | Unit formulation amount (mg/tablet) |
| Immediate-release layer | Brivaracetam | 11.31 | 25 |
| | Cross-linked carboxymethyl starch sodium | 4.52 | 10 |
| | Corn starch | 59.05 | 130.5 |
| | Anhydrous calcium hydrogen phosphate | 19.68 | 43.5 |
| | Hydroxy propyl cellulose | 3.44 | 7.6 |
| | Sodium stearyl fumarate | 1.13 | 2.5 |
| | Colloidal Silica | 0.86 | 1.9 |
| Sustained-release layer | Brivaracetam | 15.81 | 68 |
| | HPMC K4M | 20.70 | 89.025 |
| | EUDRAGIT^{®} L100-55 | 16.20 | 69.675 |
| | Anhydrous calcium hydrogen phosphate | 20.53 | 88.3 |
| | Corn starch | 24.74 | 106.4 |
| | Colloidal Silica | 0.74 | 3.2 |
| | Sodium stearyl fumarate | 1.26 | 5.4 |
| Retarding layer | HPMC K4M | 41.18 | 70 |
| | Anhydrous calcium hydrogen phosphate | 23.53 | 40 |
| | Glyceryl behenate | 26.47 | 45 |
| | Hydroxy propyl cellulose | 7.71 | 13.1 |
| | Colloidal Silica | 0.53 | 0.9 |
| | Sodium stearyl fumarate | 0.59 | 1 |
| Film coating | Opadry^{®} II 85F38197-CN Yellow | 100.00 | 25.5 |

### II. Test

### Performance test:

Dissolution rate of each stage: In vitro dissolution rate at 0.5 h, 2 h, 6 h, 14 h, 18 h after tablet dosing
Dissolution Consistency
Tablet weight Consistency
The in vitro dissolution test was conducted using the paddle method with a sinker at 50 rpm in pH 6.4 phosphate buffer.

**The following lists the factors identified during R&D that have a significant impact on the physicochemical properties of the tablets, along with the corresponding test results.**

Tables 8 to 11 present the comparative formulations used to verify contributing factors.

### Comparative Example 1

**Table 8. Formulation of Comparative Example 1**

| Layes of tablet | Raw material | Comparative Example 1 | |
|---|---|---|---|
| | | % ratio (w/w) in formulation of the layer | Unit formulation amount (mg/tablet) |
| Immediate-release layer | Brivaracetam | 8.18 | 18 |
| | Cross-linked sodium carboxylmethyl cellulose | 4.55 | 10 |
| | Lactose | 54.55 | 120 |
| | Anhydrous calcium hydrogen phosphate | 25.00 | 55 |
| | Povidone K30 | 6.00 | 13.2 |
| | Magnesium Stearate | 1.14 | 2.5 |
| | Colloidal Silica | 0.59 | 1.3 |
| Sustained-release layer | Brivaracetam | 18.67 | 40 |
| | Sodium carboxymethyl cellulose | 27.06 | 58 |
| | Anhydrous calcium hydrogen phosphate | 26.13 | 56 |
| | Microcrystalline cellulose PH101 | 26.60 | 57 |
| | Colloidal Silica | 0.47 | 1 |
| | Magnesium Stearate | 1.07 | 2.3 |
| Retarding layer | HPMC K4M | 44.12 | 75 |
| | Lactose | 22.35 | 38 |
| | Carnauba wax | 24.12 | 41 |
| | Povidone K30 | 7.35 | 12.5 |
| | Colloidal Silica | 1.06 | 1.8 |
| | Magnesium Stearate | 1.00 | 1.7 |
| Film coating | Opadry^{®} II 85F38197-CN Yellow | 100.00 | 18.6 |

In Comparative Example 1, compared with the tablet of Example 1, the sustained-release material used in the sustained-release layer was sodium carboxymethyl cellulose.

### Comparative Example 2

**Table 9. Formulation of Comparative Example 2**

| Layes of tablet | Raw accessory material | Comparative Example 2 | |
|---|---|---|---|
| | | % ratio (w/w) in formulation of the layer | Unit formulation amount (mg/tablet) |
| Immediate-release layer | Brivaracetam | 2.73 | 6 |
| | Cross-linked carboxymethyl starch sodium | 2.00 | 4.4 |
| | Corn starch | 55.00 | 121 |
| | Anhydrous calcium hydrogen phosphate | 30.91 | 68 |
| | Hydroxy propyl cellulose | 7.32 | 16.1 |
| | Sodium stearyl fumarate | 1.36 | 3 |
| | Colloidal Silica | 0.68 | 1.5 |
| Sustained-release layer | Brivaracetam | 19.73 | 44 |
| | HPMC K100M | 39.29 | 72 |
| | Anhydrous calcium hydrogen phosphate | 26.77 | 59.7 |
| | Corn starch | 19.64 | 43.8 |
| | Colloidal Silica | 0.54 | 1.2 |
| | Sodium stearyl fumarate | 1.03 | 2.3 |
| Retarding layer | Guar gum | 47.06 | 80 |
| | Anhydrous calcium hydrogen phosphate | 20.59 | 35 |
| | Carnauba wax | 23.53 | 40 |
| | Hydroxy propyl cellulose | 6.71 | 11.4 |
| | Colloidal Silica | 1.12 | 1.9 |
| | Sodium stearyl fumarate | 1.00 | 1.7 |
| Thin Film Coating | Opadry^{®} II 85F38197-CN Yellow | 100.00 | 18.6 |

In Comparative Example 2, compared with the tablet of Example 2, a ratio of the drug substance in the immediate-release layer to the total amount of drug substances in the tablet was 12%, and a ratio of the drug substance in the sustained-release layer to the total amount of drug substances in the tablet was 88%.

### Comparative Examples 3-4

**Table 10. Formulations of Comparative Examples 3-4**

| Layes of tablet | Raw accessory material | Comparative Example 3 | | Comparative Example 4 | |
|---|---|---|---|---|---|
| | | % ratio (w/w) in formulation of the layer | Unit formulation amount (mg/tablet) | % ratio (w/w) in the formulation of layer | Unit formulation amount (mg/tablet) |
| Immediate-release layer | Brivaracetam | 8.18 | 18 | 20.33 | 25 |
| | Cross-linked carboxymethyl starch sodium | 5.00 | 11 | 8.94 | 11 |
| | Corn starch | 54.41 | 119.7 | 29.02 | 35.7 |
| | Anhydrous calcium hydrogen phosphate | 25.91 | 57 | 30.08 | 37 |
| | Hydroxy propyl cellulose | 5.00 | 11 | 8.94 | 11 |
| | Sodium stearyl fumarate | 1.00 | 2.2 | 1.79 | 2.2 |
| | Colloidal Silica | 0.50 | 1.1 | 0.89 | 1.1 |
| Sustained-release layer | Brivaracetam | 21.31 | 52 | 19.70 | 52 |
| | HPMC K4M | 23.91 | 58.35 | 22.10 | 58.35 |
| | EUDRAGIT^{®} L100-55 | 16.17 | 39.45 | 14.94 | 39.45 |
| | Anhydrous calcium hydrogen phosphate | 16.56 | 40.4 | 19.09 | 50.4 |
| | Corn starch | 20.64 | 50.35 | 22.86 | 60.35 |
| | Colloidal Silica | 0.47 | 1.15 | 0.44 | 1.15 |
| | Sodium stearyl fumarate | 0.94 | 2.3 | 0.87 | 2.3 |
| Retarding layer | Guar gum | 42.41 | 72.1 | 42.41 | 72.1 |
| | Anhydrous calcium hydrogen phosphate | 21.18 | 36 | 21.18 | 36 |
| | Carnauba wax | 24.41 | 41.5 | 24.41 | 41.5 |
| | Hydroxy propyl cellulose | 10.00 | 17 | 10.00 | 17 |
| | Colloidal Silica | 1.00 | 1.7 | 1.00 | 1.7 |
| | Sodium stearyl fumarate | 1.00 | 1.7 | 1.00 | 1.7 |
| Thin Film Coating | Opadry^{®} II 85F38197-CN Yellow | 100.00 | 18.6 | 100.00 | 18.6 |

In Comparative Example 3, compared with the tablet of Example 3, a ratio of the drug substance in the sustained-release layer to the total formulation amount of the sustained-release layer is greater than 20%; and in Comparative Example 4, compared with the tablet of Example 3, a ratio of the drug substance in the immediate-release layer to the total formulation amount of the immediate-release layer is greater than 20%.

### Comparative Examples 5-6

**Table 11. Formulations of Comparative Examples 5-6**

| Layes of tablet | Raw accessory material | Comparative Example 5 | | Comparative Example 6 | |
|---|---|---|---|---|---|
| | | % ratio (w/w) in the formulation of layer | Unit formulation amount (mg/tablet) | % ratio (w/w) in the formulation of layer | Unit formulation amount (mg/tablet) |
| Immediate-release layer | Brivaracetam | 12.84 | 28 | 12.84 | 28 |
| | Cross-linked carboxymethyl starch sodium | 6.88 | 15 | 6.88 | 15 |
| | Corn starch | 46.24 | 100.8 | 46.24 | 100.8 |
| | Anhydrous calcium hydrogen phosphate | 28.58 | 62.3 | 28.58 | 62.3 |
| | Hydroxy propyl cellulose | 3.94 | 8.6 | 3.94 | 8.6 |
| | Sodium stearyl fumarate | 1.01 | 2.2 | 1.01 | 2.2 |
| | Colloidal Silica | 0.50 | 1.1 | 0.50 | 1.1 |
| Sustained-release layer | Brivaracetam | 16.11 | 72 | 16.11 | 7 |
| | HPMC K100M | 20.13 | 90 | 20.13 | 90 |
| | EUDRAGIT^{®} L100-55 | 6.71 | 30 | 6.71 | 30 |
| | Anhydrous calcium hydrogen phosphate | 25.77 | 115.2 | 25.77 | 115.2 |
| | Corn starch | 29.17 | 130.4 | 29.17 | 130.4 |
| | Colloidal Silica | 1.07 | 4.8 | 1.07 | 4.8 |
| | Sodium stearyl fumarate | 1.03 | 4.6 | 1.03 | 4.6 |
| Retarding layer | HPMC K4M | 54.12 | 92.00 | 29.41 | 50 |
| | Anhydrous calcium hydrogen phosphate | 26.06 | 44.3 | 43.12 | 73.3 |
| | Carnauba wax | 10.00 | 17.00 | 17.65 | 30 |
| | Hydroxy propyl cellulose | 8.41 | 14.3 | 8.41 | 14.3 |
| | Colloidal Silica | 0.71 | 1.2 | 0.71 | 1.2 |
| | Sodium stearyl fumarate | 0.71 | 1.2 | 0.71 | 1.2 |
| Thin Film Coating | Opadry^{®} II 85F38197-CN Yellow | 100.00 | 25.50 | 100.00 | 25.5 |

In Comparative Example 5, compared with the tablet of Example 4, a ratio of wax (carnauba wax) in the retarding layer to HPMC by weight was 0.18; and in Comparative Example 6, compared with the tablet of Example 4, the total amount of the wax (carnauba wax) + HPMC in the retarding layer was 47.06%.

### Comparative Example 7

Comparative Example 7 differs from Example 5 in that the total granules of the retarding layer prepared in Example 5 were used in Comparative Example 7, with the retarding layer compressed to a thickness of 0.8 mm, while the rest of the formulation was identical to that of Example 5.

### Comparative Example 8

The difference between Comparative Example 8 and Examples 5, 6 and 7 was that a ratio of microcrystalline cellulose to lactose in the immediate-release layer of Comparative Example 8 was 1:4, and the others were consistent with those of Examples 5, 6 and 7.

### 2.1 Effects of sustained-release materials

Comparative Example 1 differed from Example 1 only in the sustained-release material.

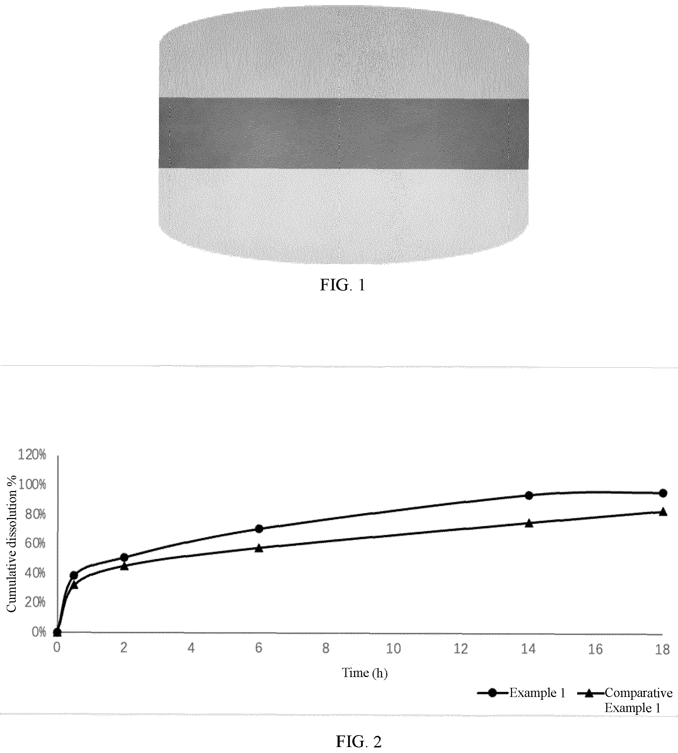

The dissolution curves of brivaracetam in Example 1 and Comparative Example 1 were shown in FIG. 2.

Dissolution characteristics: As shown in FIG. 2, after it was replaced with sodium carboxymethyl cellulose, the dissolution rate in each stage decreased, especially in Comparative Example 1, which was significantly lower than that in Example 1 after 6 hours, which led to the low plasma concentration in the sustained-release stage and made it difficult to achieve the drug effect.

In Example 1, a dissolution rate of a drug substance was no less than 25% at half an hour, 30-65% at 2 hours, no less than 65% at 6 hours, and no less than 80% but less than 100% at 14 hours after tablet dosing; and the tablet was released in zero order half an hour after tablet dosing.

### 2.2 Effect of distribution ratio of API in sustained-release layer and immediate-release layer

The difference between Comparative Example 2 and Example 2 was only that the ratio of API in the immediate-release layer and the sustained-release layer was different, and other accessory materials were completely the same. In the Comparative Example 2: The ratio of the drug substance in the immediate-release layer to the total amount of drug substances in the tablet was 12% (less than 20%), and the ratio of the drug substance in the sustained-release layer to the total amount of drug substances in the tablet was 88% (higher than 80%).

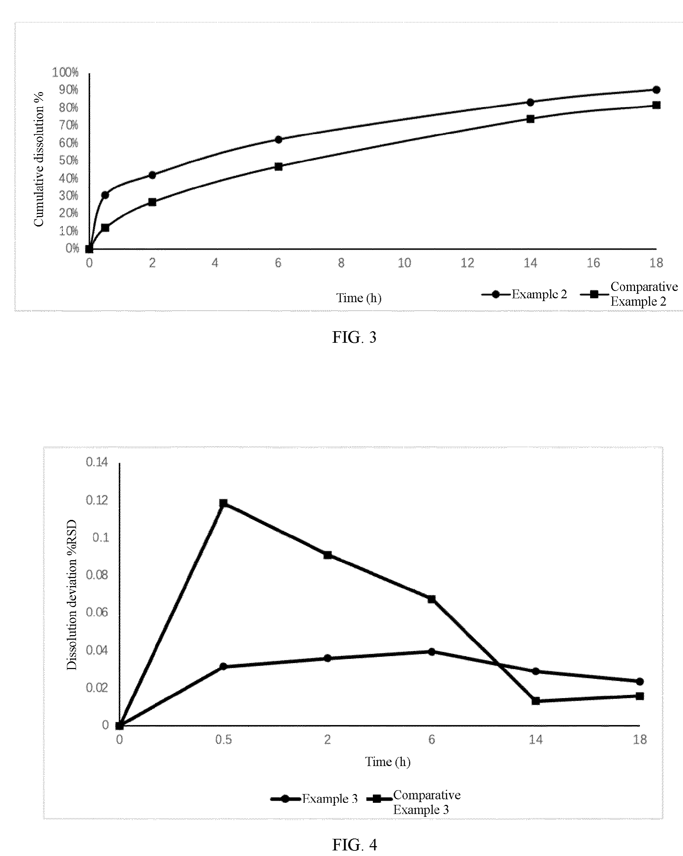

The dissolution curves of brivaracetam in Example 2 and Comparative Example 2 were shown in FIG. 3.

Dissolution characteristics: As shown in FIG. 3, after the API in the immediate-release layer was reduced, it reached more than 25% after 2 hours of tablet dosing, which could not play a role in rapid onset of drug dosing.

However, in Example 2, the dissolution rate of the drug substance in the tablet 2 was 30% at half an hour, 30-60% at 2 hours, no less than 65% at 6 hours, and no less than 80% but less than 100% at 14 hours; and the tablet was released in zero order half an hour after tablet dosing.

### 2.3 Effect of API content in the sustained-release layer

Comparative example 3 differed from Example 3 only in that: in terms of the ratio of API to accessory materials in the sustained-release layer, in Comparative Example 3 the content of the filler was reduced, so that the ratio of API content was increased from 19.7% to 21.31%, which was slightly higher than 20%.

6 tablets of each of Example 3 and Comparative Example 3 were randomly selected for dissolution experiments. The dissolution data of Example 3 was shown in Table 12 below, and the dissolution data of Comparative Example 3 was shown in Table 13 below.

**Table 12. Example 3, with a paddle method, at 50 rpm, at pH 6.4, 900 ml, n = 6 tablets, dissolution and dissolution deviation**

| % dissolution of brivaracetam - time point (h) | 0.0 | 0.50 | 2.00 | 6.00 | 14.00 | 18.00 |
|---|---|---|---|---|---|---|
| Tablet 1 | 0.0 | 42.2 | 57.7 | 74.7 | 93.0 | 94.6 |
| Tablet 2 | 0.0 | 41.0 | 58.3 | 78.3 | 97.7 | 98.9 |
| Tablet 3 | 0.0 | 40.6 | 56.3 | 79.0 | 98.0 | 99.8 |
| Tablet 4 | 0.0 | 40.5 | 61.4 | 81.2 | 97.2 | 98.3 |
| Tablet 5 | 0.0 | 44.0 | 61.6 | 80.1 | 97.7 | 99.3 |
| Tablet 6 | 0.0 | 41.8 | 59.9 | 84.2 | 101.9 | 101.7 |
| **%RSD** | **0.0** | **3.15** | **3.60** | **3.95** | **2.90** | **2.37** |

**Table 13. Comparative Example 3, with a paddle method, at 50 rpm, at pH 6.4, 900 ml, n = 6 tablets, dissolution and dissolution deviation**

| % dissolution of brivaracetam - time point (h) | 0.0 | 0.50 | 2.00 | 6.00 | 14.00 | 18.00 |
|---|---|---|---|---|---|---|
| Tablet 1 | 0.0 | 44.1 | 65.4 | 86.4 | 103.3 | 105.5 |
| Tablet 2 | 0.0 | 48.4 | 66.2 | 90.3 | 107.4 | 109.0 |
| Tablet 3 | 0.0 | 49.6 | 71.3 | 96.7 | 104.4 | 104.2 |
| Tablet 4 | 0.0 | 48.1 | 68.2 | 95.3 | 104.1 | 104.8 |
| Tablet 5 | 0.0 | 45.8 | 64.6 | 88.6 | 105.1 | 106.1 |
| Tablet 6 | 0.0 | 60.7 | 81.5 | 103.5 | 104.7 | 106.8 |
| **%RSD** | **0.0** | **11.85** | **9.11** | **6.75** | **1.32** | **1.59** |

At the same time, the dissolution deviation curve of two kinds of tablets was shown in FIG. 4.

As shown in Table 13, none of the tablets in Comparative Example 3 could meet the requirements of the present disclosure: a dissolution rate of a drug substance was no less than 25% at half an hour, 30-65% at 2 hours, no less than 65% at 6 hours, and no less than 80% but less than 100% at 14 hours after tablet dosing; and the tablet was released in zero order half an hour after tablet dosing.

As shown in FIG. 4, it could be seen that the dissolution deviation RSD of Comparative Example 3 was relatively large.

It could be determined that the absolute content of the API in the sustained-release layer remained unchanged, but when the content of the filler decreased, the dissolution characteristics of the tablets would be significantly affected, and the plasma concentration would be affected accordingly. That was, the drug effectiveness, stability and safety would be affected.

### 2.4 Effect of API content in the immediate-release layer

The only difference between Comparative Example 4 and Example 3 was that the ratio of the API in the formulation of the immediate-release layer of Comparative Example 4 was higher than 20%.

The test showed that the tablet weight consistency was obviously lower than that of Example 3.

**Table 14.**

| | | |
|---|---|---|
| Difference in the dissolution tablet weight of the three-layered tablet for the brivaracetam sustained-release tablet | 10 tablets were compressed continuously, and the difference of tablet weight was compared | |
| | % difference in tablet weight of Example 3 | % difference in tablet weight of Comparative Example 4 |
| | 0.24 | 6.80 |

In the present disclosure, it was found that as shown in the formulations of Comparative Example 4 and Example 3 and Table 14, the layer weight was smaller and the tablet weight consistency was poor when the API content in the immediate-release layer was higher.

### 2.5 Effect of composition ratio of sustained-release materials in retarding layer

The only difference between Comparative Example 5 and Example 4 was that the ratio of the waxy sustained-release material to the swelling-type sustained-release material among the sustained-release materials in the retarding layer was lower than 0.5.

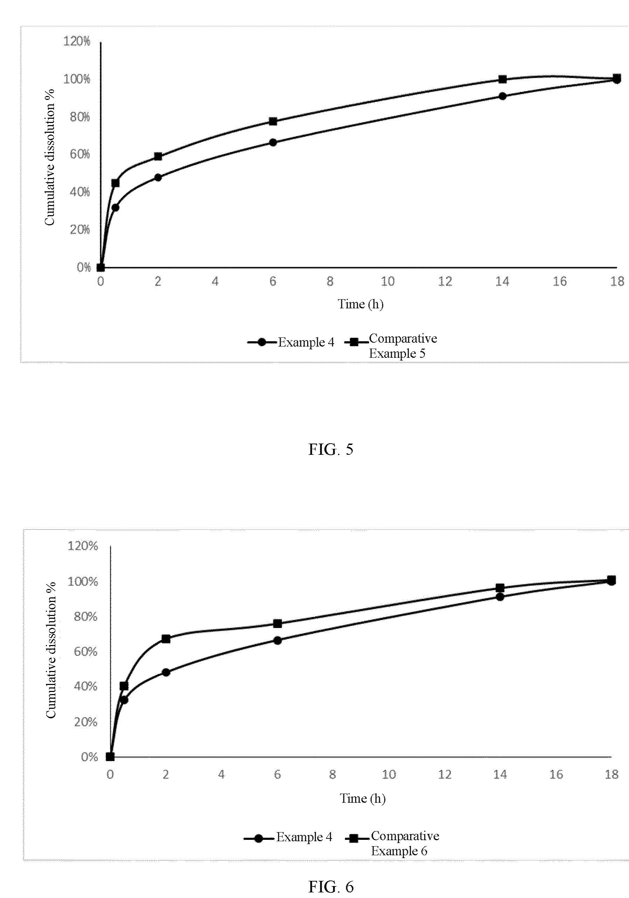

The dissolution data was shown in FIG. 5.

As shown in FIG. 5, when the ratio of the waxy sustained-release material to the swelling-type sustained-release material among the sustained-release materials in the retarding layer was reduced, the most obvious effect was that the dissolution ratio in each stage was significantly higher than that in the tablet of Example 4, which would lead to higher plasma concentration in the early stage and reduced drug action time at the same time.

### 2.6 Effect of the content of the sustained-release material in the retarding layer

The only difference in tablets between Comparative Example 6 and Example 4 was that the ratio of the sustained-release material in the retarding layer was lower than 60% (the ratio in Example 4 was 64.12%). That was, the ratios of the filler and other accessory materials were increased.

The dissolution data was shown in FIG. 6.

As shown in FIG. 6, when the total ratio of the sustained-release material in the retarding layer was reduced, the dissolution ratio in each stage was significantly higher than that in the tablet of Example 4, and the dissolution curve tended to be flat after 3 hours, which would lead to a high plasma concentration in the early stage of drug release, and then the plasma concentration could not reach an effective level, which could not really take into account rapid onset and long-term effectiveness, and actually the effective time was shortened.

### 2.7 Effect of the thickness of the retarding layer

The difference between Comparative Example 7 and Example 5 was that the thickness of the retarding layer in Comparative Example 7 was 0.08 mm, and the thickness of the retarding layer in Example 5 was 2 mm.

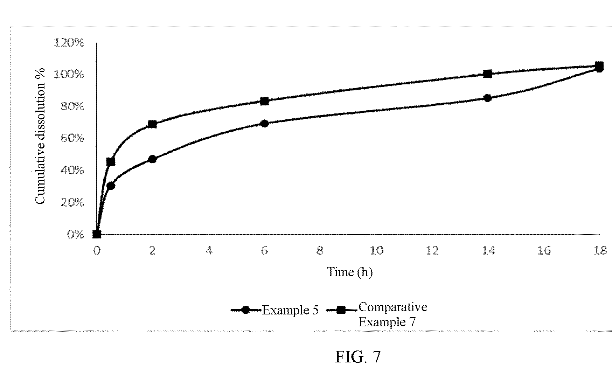

The test results were shown in FIG. 7:
As shown in Figure 7, reducing the mass proportion of the retarding layer (i.e., decreasing its thickness) primarily affects the dissolution characteristics of the tablet by causing excessively rapid release, which may result in an overly high plasma drug concentration in the early stage and a shortened duration of efficacy.**2.8 Effect of the ratio of microcrystalline cellulose to lactose in the**

### immediate-release layer

Comparative Example 8 differed from Examples 5, 6 and 7 in that the ratio of microcrystalline cellulose to lactose in the immediate-release layer was different, but the total amount of the microcrystalline cellulose and the lactose in the immediate-release layer remained unchanged. The ratio of microcrystalline cellulose to lactose in Comparative Example 8 was 1:4, the ratio of microcrystalline cellulose to lactose in Example 5 was 1:2, the ratio of microcrystalline cellulose to lactose in Example 6 was 1:1, and the ratio of microcrystalline cellulose to lactose in Example 7 was 1:3.

The flowability of granules in the immediate-release layer was shown in Table 15:

**Table 15.**

| | Example 5 | Example 6 | Example 7 | Comparative Example 8 |
|---|---|---|---|---|
| Repose angle° | 36.9 | 36 | 35.3 | 44.7 |
| Hausner ratio | 1.16 | 1.13 | 1.22 | 1.41 |

The acute angle formed between the slope of a powder heap and the horizontal plane at its base is referred to as the angle of repose. A larger angle of repose indicates a higher friction coefficient and poorer powder flowability Generally, powders are considered to have good flowability when the angle of repose is less than 40°. The Hausner ratio is the ratio of tapped density to bulk density of a powder. A higher Hausner ratio indicates poorer flowability, and a Hausner ratio greater than 1.35 suggests poor flow characteristics. As shown in As shown in Table 15, the powder flowability of the granules in the immediate-release layer of Comparative Example 8 was poor, with an angle of repose of 44.7°, exceeding 40°, and a Hausner ratio of 1.41, exceeding 1.35. In contrast, the powders in Examples 5, 6, and 7 met the acceptable standards.

## Claims

1. A double-release three-layered brivaracetam tablet, which is made of an immediate-release layer, a retarding layer and a sustained-release layer that are superposed; wherein
the retarding layer contains no drug and is located in the middle;
at least 60% of the surface area of each of the immediate-release layer and the sustained-release layer that is in contact with the retarding layer is covered by the retarding layer; and
a sustained-release material in the retarding layer contains a waxy sustained-release material; drug substance is selected from one or more of brivaracetam, a pharmaceutically acceptable complex thereof, a pharmaceutically acceptable salt thereof, a pharmaceutically acceptable solvate thereof, a pharmaceutically acceptable hydrate thereof, and other related structural derivatives of levetiracetam;
the tablet has the following characteristics: when tested using the paddle method at 50 rpm, in medium of pH 1.2, pH 4.5, pH 6.8, or water in an aqueous medium, a dissolution rate of the drug substance is not less than 25% at 0.5 hour, from 30% to 65% at 2 hours, not less than 65% at 6 hours, and not less than 80% but less than 100% at 14 hours; and after 0.5 hour, the tablet is released in vitro in a zero-order release manner.

2. The double-release three-layered brivaracetam tablet according to claim 1, wherein the content of the drug substance in the immediate-release layer accounts for 20 to35% or 25 to30% of the total amount of the drug substance; and the content of the drug substance in the sustained-release layer account for 65-80% or 70-75% of the total amount of the drug substance.

3. The double-release three-layered brivaracetam tablet according to claim 1, wherein the formulation of the immediate-release layer comprises:
a disintegrant in an amount of 2% to 7.5%, or preferably 5%, by weight of the immediate-release layer, wherein the disintegrant is selected from one or more of crosslinked sodium carboxymethyl starch, crosslinked sodium carboxymethyl cellulose, cross-linked povidone, and low-substituted hydroxypropyl cellulose;
a filler in an amount of 40% to 85% by weight of the immediate-release layer, wherein the filler is selected from one or more of lactose, microcrystalline cellulose, anhydrous dibasic calcium phosphate, and corn starch; and; and
an adhesive in an amount of 3% to 8% by weight of the immediate-release layer, wherein the adhesive is selected from hydroxypropyl cellulose and an aqueous solution of povidone K30.

4. The double-release three-layered brivaracetam tablet according to claim 3, wherein the filler is a combination of anhydrous calcium hydrogen phosphate and corn starch, or a combination of microcrystalline cellulose and lactose.

5. The double-release three-layered brivaracetam tablet according to claim 4, wherein in the combination of microcrystalline cellulose and lactose, the weight ratio of the two components is from 1:1 to 1:3.

6. The double-release three-layered brivaracetam tablet according to claim 1, wherein the waxy sustained-release material is selected from one or more of glyceryl behenate, carnauba wax and cetyl alcohol.

7. The double-release three-layered brivaracetam tablet according to claim 1, wherein the sustained-release material in the retarding layer consists of a waxy sustained-release material and a swelling-type sustained-release material, wherein the weight ratio of the waxy sustained-release material to the swelling-type sustained-release material is 0.4 to 0.8 or 0.5 to 0.7; and the combined content of the two materials accounts for not less than 60% by weight of the formulation of the retarding layer;
wherein the swelling-type sustained-release material is selected from one or more of guar gum, vegetable gum, seaweed glue, hydroxypropyl methylcellulose, hydroxymethyl cellulose and hydroxyethyl cellulose.

8. The double-release three-layered brivaracetam tablet according to claim 7, wherein a thickness of the retarding layer is greater than 1 mm or equal to 1mm to 3 mm.

9. The double-release three-layered brivaracetam tablet according to claim 8, wherein the filler is present in an amount of 15% to 30% or 20% to 27%, by weight of the retarding layer, and is selected from lactose, microcrystalline cellulose, anhydrous calcium hydrogen phosphate or mannitol; and
the adhesive is present in an amount of 6% to15% by weight of the retarding layer, and is selected from hydroxypropyl cellulose or a povidone K30 aqueous solution.

10. The double-release three-layered brivaracetam tablet according to claim 1, wherein a formulation of the sustained-release layer comprises the drug substance , a sustained-release material, a filler, a flow aid and a lubricant;
wherein the sustained-release material is present in an amount of 20% to60% or 25% to 40% by weight of the formulation ; and
the sustained-release material is selected from one or two of HPMC K15M, HPMC K4M, HPMC K750, HPMC K100M, HPMC K100LV, EUDRAGIT^{®} L100-55, glyceryl behenate and xanthan gum.

11. The double-release three-layered brivaracetam tablet according to claim 10, wherein the sustained-release material is a combination of HPMC K100M and EUDRAGIT^{®} L100-55, or a combination of HPMC K4M and EUDRAGIT^{®} L100-55, or HPMC K100M; and
the filler is present in an amount of 35% to65% by weight of the sustained-release layer, and selected from one or more of microcrystalline cellulose, lactose, anhydrous calcium hydrogen phosphate, corn starch and the like.

12. The double-release three-layered brivaracetam tablet according to any one of claims 1-11, wherein in the formulation of the immediate-release layer or the formulation of the sustained-release layer, the content of the drug substance is no higher than 20%.

13. The double-release three-layered brivaracetam tablet according to any one of claims 1-11, further comprise a film coating, the coating material being a gastric-soluble coating powder, and the weight gain from the film coating is 2% to 5%, or 3%.

14. A method for preparing the double-release three-layered brivaracetam tablet according to any one of claims 1-13, comprising the following steps:
step a. weigh the brivaracetam, disintegrant, and filler in amounts required for the formulation of the immediate-release layer, and mix them thoroughly; add an adhesive to the mixture and perform wet granulation, after wet sizing, dry the granules until the moisture content is less than 4.0%, then carry out dry sizing and final blending to obtain the final granules of the immediate-release layer;
step b. weigh a sustained-release material and a filler in amounts required for the formulation of a sustained-release layer, and mix them uniformly; add a proper amount of purified water by spray addition for wet granulation, after wet sizing, dry the granules until the moisture content is less than 4.0%, then perform dry sizing and final blending to obtain the final granules of the sustained-release layer; and
step c. weigh a sustained-release material and a filler in amounts required for the formulation of the retarding layer, and mix them uniformly; add an adhesive, and perform wet granulation. After wet sizing, dry the granules until the moisture content is less than 4.0%, then perform dry sizing and final blending to obtain the final granules of the retarding layer.

15. The preparation process according to claim 14, wherein the final blending in the Step a comprises: mixing a portion of the dried granules with a glidant in amounts required for the formulation, followed by sieving; pre-mixing the resulting blend with the remaining dried granules of the immediate-release layer; then mixing a portion of the pre-mixture with a lubricant in amounts required for the formulation, followed by sieving; and then blending with the remaining pre-mixture to obtain the final granules of the immediate-release;;
the final blending in the Step b comprises: mixing a portion of the dried granules with a glidant in amounts required for the formulation, followed by sieving; pre-mixing the resulting blend with the remaining dried granules of the sustained-release layer; mixing a portion of the pre-mixture a lubricant in amounts required for the formulation, followed by sieving; and then blending with the remaining pre-mixture to obtain the final granules of the sustained-release layer; and
the final blending in the step c comprises: mixing a portion of the dried granules with glidant in amounts required for the formulation, followed by sieving; pre-mixing the resulting blend with the remaining dried granules of the retarding layer; mixing a portion of the pre-mixture with a lubricant in amounts required for the formulation, followed by sieving; and then blending with the remaining pre-mixture to obtain the final granules of the retarding layer.

16. The preparation process according to claim 14, further comprising a tableting step, wherein a tableting sequence is the sustained-release layer, the retarding layer and the immediate-release layer.
